# EUROPÄISCHE PATENTANMELDUNG

(11) **EP 1 350 788 A2**
(43) Veröffentlichungstag der Anmeldung: **08.10.2003**
(21) Anmeldenummer: 02007121.3
(22) Anmeldetag: 28.03.2002
(51) Int. Cl.: C07C 209/48, C07C 253/10

(54) **Verfahren zur Herstellung von Hexamethylendiamin aus Butadien**

(71) Anmelder: Degussa AG, 40474 Düsseldorf (DE)
(72) Erfinder: Brasse, Claudia, Dr., 63452 Hanau (DE); Haas, Thomas, Dr., 60322 Frankfurt (DE); Weber, Robert, Dr., 53879 Euskirchen (DE); Neuroth, Jürgen, 60388 Frankfurt (DE)

(57) **Zusammenfassung**

Die Erfindung richtet sich auf die Herstellung von Hexamethylendiamin aus Butadien. Das Verfahren umfasst die hintereinander durchgeführten Stufen (i) einer katalytischen Epoxidation von Butadien zu 1,2-Epoxy-3-buten, (ii) einer basischn katalysierten Addition von Cyanwasserstoff an Butadienmonoxid unter Bildung eines 3-Hydroxy-4-pentennitril (3HPN) und 2-Hydroxymethyl-3-butennitril (2HBN) enthaltenden Reaktionsgemischs, (iii) einer sauer katalysierten Dehydratisierung der Cyanhydride 3HPN und 2HMBN der Stufe (ii) zu cis/trans-Pentadienitril (PDN), (iv) einer basisch katalysierten Addition der Stufe (iii) unter Bildung von cis/trans-1,4-Dicyanebuten-1 und-2 (DCB) und (v) einer katalytischen Hydrierung der isomeren cis/trans-1,4-Dicyanobutene der Stufe (iv) zu Hexamethylendiamin.

## Beschreibung

Die Erfindung richtet sich auf ein mehrstufiges Verfahren zur Herstellung von Hexamethylendiamin (HMDA) aus Butadien, wobei in der ersten Stufe Butadien in 1,2-Epoxy-3-buten (Butadienmonoxid) überführt wird und das weitere Verfahren zwei Cyanwasserstoff-Additionsstufen, eine Dehydratisierungsstufe und eine Hydrierstufe umfasst.

Hexamethylendiamin ist ein in großtechnischer Menge produziertes Zwischenprodukt, wofür ein weiterer Zuwachs prognostiziert wird. Bekannte Rohstoffe zur Herstellung von Hexamethylendiamin sind einerseits Cyclohexan und andererseits Butadien. Ein Verfahren zur Herstellung von HMDA aus Cyclohexan umfasst eine Oxidation desselben zu Adipinsäure, eine Veresterung der Adipinsäure, eine nachfolgende Hydrierung zu 1,6-Hexandiol und Umsetzung desselben mit Ammoniak und Bildung HMDA. Gemäß einem alternativen Verfahren wird die aus Cyclohexan oder Cyclohexen gewonnene Adipinsäure durch Aminierung und Hydrierung zunächst in Adiponitril und letzteres durch katalytische Hydrierung in Hexamethylendiamin überführt. Ein weiteres Verfahren basiert auf Acrylnitril, das elektrochemisch dimerisiert und wobei das erhaltende Adiponitril hydriert wird.

Unter Verwendung von Butadien als Rohstoff lässt sich HMDA gewinnen, indem Butadien mittels Sauerstoff und Chlorwasserstoff zunächst in ein Dichlorbuten-Isomerengemisch überführt, dieses mit einem Alkalicyanid zu einem Dicyanbuten-Isomerengemisch umgesetzt und letzteres über die Zwischenstufe Adiponitril zu Hexamethylendiamin hydriert wird. Der hohe Salzanfall macht dieses Verfahren für einen großtechnischen Einsatz wenig attraktiv.

Gemäß einer alternativen Syntheseroute zur Herstellung von Hexamethylendiamin aus Butadien wird Butadien in Gegenwart eines Nickelkatalysators mit einem Phosphin-/ Phosphit-Liganden mit Cyanwasserstoff umgesetzt, wobei 1-Cyano-buten-2 und 3-Cyano-buten-1 gebildet werden. Dieses Isomerengemisch wird durch eine 2-stufige Isomerisierung in Gegenwart verschiedener Nickelkatalysatoren in 1-Cyano-Buten-3 (1-Pentennitril) überführt. Durch Cyanwasserstoffaddition an das 1-Cyano-Buten-3 in Gegenwart eines homogenen Nickelkatalysators wird Adiponitril gebildet, woraus durch katalytische Hydrierung Hexamethylendiamin entsteht.

Eine Übersicht über die derzeit bekannten und technisch genutzten Verfahren zur Herstellung von Hexamethylendiamin sind Chemical Economics Handbook - SRI International, February 2000, Nr. 664.1000 G bis J, zu entnehmen. Ein wesentlicher Nachteil des bisher genutzten Verfahrens zur Herstellung von Hexamethylendiamin aus Butadien besteht darin, dass in mehreren Stufen homogene Nickelkatalysatoren eingesetzt werden müssen, deren Regenerierung aufwendig ist und zudem ein erheblicher Aufwand erforderlich ist, um arbeitshygienische Probleme zu vermeiden.

Das US-Patent 2,488,913 lehrt ein Verfahren zur Herstellung von 1,4-Dicyanobuten, das seinerseits zu Hexamethylendiamin hydriert werden kann. Gemäß einem Beispiel dieses Dokuments wird 1,2-Epoxybuten-3 in Gegenwart eines Katalysatorsystems aus Kupfer(I)chlorid, Kupfer, Chlorwasserstoff und Ammoniumchlorid in wässriger Phase mit Cyanwasserstoff umgesetzt, wobei 1,4-Dicyanobuten gebildet wird.

Die Erfinder der vorliegenden Anmeldung haben das in der US 2,488,913 beschriebene Verfahren nachgearbeitet und dabei festgestellt, dass das gewünschte 1,4-Dicyanobuten-2 nur in geringem Umfang, nämlich laut gaschromatographischer Analyse mit 8 Flächen-Prozent gebildet wird. Auch das in diesem Dokument beschriebene Beispiel 3, wonach die Cyanwasserstoffaddition unter der zuvor genannten Katalyse an aus Butadienmonoxid zugängliches 1-Cyano-2-hydroxybuten-3 erfolgt, führte zu keinem besseren Ergebnis. Schließlich wurde auch festgestellt, dass das in diesem Dokument angegebene Verfahren zur Herstellung der Vorstufe 1-Cyano-2-hydroxybuten-3 durch Cyanwasserstoff-Addition an 1,2-Epoxybuten-3 in wässriger Phase in Gegenwart von Kalciumhydroxid als Katalysator unter den in diesem Dokument angegebenen Bedingungen nicht gebildet wird.

Das US-Patent 2,473,486 lehrt ein Verfahren, zur Herstellung von 1-Cyano-1,3-Butadien (Pentadiennitril), das eine Addition von Cyanwasserstoff an 1,2-Epoxy-3-buten umfasst. Gemäß dem in diesem Dokument beschriebenen Verfahren wird Cyanwasserstoff durch Umsetzung von Natriumcyanid mit Kohlendioxid im Reaktionsgemisch in-situ gebildet. Das Reaktionsgemisch enthält außer einer kleinen Menge Pentadiennitril 1-Cyano-3-buten-2- (= 3-Hydroxy-4-pentennitril). Das Reaktionsgemisch wird mittels eines Anhydrids azyliert und das so erhaltene Reaktionsgemisch bei 350 - 700 °C pyrolysiert, wobei Pentadiennitril gebildet wird. Hinweise zur Verwendung des Pentadiennitrils zur Herstellung von Hexamethylendiamin lassen sich diesem Dokument nicht entnehmen.

Aufgabe der vorliegenden Erfindung ist es, ein weiteres Verfahren zur Herstellung von Hexamethylendiamin aus Butadien aufzuzeigen. Das Verfahren sollte wie das bekannte, technisch genutzte Verfahren nicht mehr als 5 Stufen aufweisen. Gemäß einer weiteren Aufgabe sollte auf die Verwendung eines homogenen Nickelkatalysators für die Cyanwasserstoffaddition an ein olefinisches Substrat verzichtet werden. Gemäß einer weiteren Aufgabe sollten solche Katalysatorsysteme für die HCN-Addition aufgezeigt werden, womit hohe Selektivitäten der entsprechenden Additionsprodukte erzielt werden können.

Ein weiterer Gegenstand der vorliegenden Erfindung richtet sich auf ein Verfahren zur Herstellung von Hexamethylendiamin aus 1,2-Epoxy-3-buten (Butadienmonoxid), wobei die aus dem US-Patent 2,488,913 resultierenden Probleme überwunden werden sollten.

Diese und weitere Aufgaben, wie sie sich aus der weiteren Beschreibung ergeben, lassen sich durch das erfindungsgemäße Verfahren lösen. Gefunden wurde demgemäß ein Verfahren zur Herstellung von Hexamethylendiamin, gekennzeichnet durch die hintereinander durchgeführten Stufen (i) einer katalytischen Epoxidation von Butadien zu 1,2-Epoxy-3-buten (Butadienmonoxid), (ii) einer basisch katalysierten Addition von Cyanwasserstoff an Butadienmonoxid unter Bildung eines 3-Hydroxy-4-pentennitril (3HPN) und 2-Hydroxymethyl-3-butennitril (2HMBN)enthaltenden Reaktionsgemischs , (iii) einer sauer katalysierten Dehydratisierung der Cyanhydrine 3HPN und 2HMBN der Stufe (ii) zu cis/trans-Pentadiennitril (PDN), (iv) einer basisch katalysierten Addition von Cyanwasserstoff an cis/trans-Pentadiennitril der Stufe (iii) unter Bildung von cis/trans-1,4-Dicyanobuten-1 und -2 (DCB) und (v) einer katalytischen Hydrierung der isomeren cis/trans-1,4-Dicyanobuten der Stufe (iv) zu Hexamethylendiamin.

Die Unteransprüche richten sich auf bevorzugte Ausführungsformen des erfindungsgemäßen Verfahrens.

Bei der Reaktionsstufe (i) handelt es sich um eine bekannte Epoxidation von 1,3-Butadien. Beispielsweise lässt sich Butadien durch eine selektive Gasphasepoxiation unter Verwendung von Sauerstoff in Gegenwart eines basischen Katalysators oder eines Silberkatalysators epoxidieren - beispielhaft für derartige Epoxidationen werden die US-Patente 6,172,245, 6,018,061 und 5,756,779 genannt. Diese erste Stufe der erfindungsgemäßen Verfahrenssequenz lässt sich somit in an sich bekannter Weise durchführen, Butadien ist auch im Handel erhältlich.

Die Anlagerung von Cyanwasserstoff an Butadienmonoxid in Gegenwart eines basischen Katalysators lässt sich vorzugsweise in Gegenwart eines Katalysators aus der Reihe der Alkalihydroxide, Alkalicyanide, Erdalkalihydroxide, ausgenommen Calciumhydroxid in wässriger Phase, Erdalkalicyanide, basischen Ionenaustauscher in der OH-Form und carbocyclischen sekundären oder tertiären Amine. Gemäß besonders bevorzugter Ausführungsformen der Stufe (ii) wird als Katalysator Lithiumhydroxid, in-situ gebildetes Lithiumcyanid oder ein N-alkyliertes cyclisches Amin mit 5 oder 6 Ringgliedern, wobei Alkyl für Methyl, Ethyl, n-Propyl oder n-Butyl steht und der Ring zusätzlich ein weiteres Heteroatom enthalten kann, verwendet.

Die erfindungsgemäße basekatalysierte HCN-Addition der Stufe (ii) erfolgt chemoselektiv an der Epoxidfunktion und mit hoher Regioselektivität am C-1-Atom. Die Bildung von 3-Hydroxy-4-Pentennitril (3-HPN) gegenüber 2-Hydroxymethyl-3-butennitril (2-HMBN) ist somit bevorzugt.

Die HCN-Addition an Butadienmonoxid erfolgt bevorzugt in einem organischen Lösungsmittel, das jedoch auch wasserhaltig sein kann. Es wurde gefunden, dass sich die HCN-Addition mit hoher Regioselektivität bezüglich des Verhältnisses von 3-HPN zu 2-HMBN durchführen lässt, wenn die Umsetzung in Gegenwart eines aprotischen Lösungsmittels, wie Acetonitril, Dimethylformamid, N-Methyl-pyrrolidon durchgeführt wird. Zweckmäßigerweise erfolgt die HCN-Addition bei erhöhter Temperatur insbesondere bei einer Temperatur, im Bereich von 50 - 150 °C.

Gemäß einer besonders bevorzugten Ausführungsform der HCN-Addition an Butadienmonoxid wird als Katalysator eine wässrige Lösung Lithiumhydroxid verwendet und die Umsetzung bei einer Temperatur im Bereich von 70 - 120 °C in Gegenwart von Dimethyformamid als Lösungmittel durchgeführt. Anstelle des wässrigen Lithiumhydroxids kann als Katalysator auch ein in-situ gebildetes Lithiumcyanid in gelöster oder suspendierter Form verwendet werden.

Das die Cyanhydrine 3-HPN und 2-HMBN enthaltende Reaktionsgemisch oder die aus diesem in an sich bekannter Weise isolierten Cyanhydine 3-HPN und 2-HMBN werden der Dehydratisierungsstufe (iii) zugeführt. Hierzu wird soweit anwesend, der basische Katalysator neutralisiert oder entfernt und durch einen sauren Dehydratisierungskatalysator ersetzt. Zweckmäßigerweise wird die Dehydratisierung der Cyanhydrine in einem organischen Lösungsmittel, womit gleichzeitig gebildetes Wasser als Azeotrop abgeschleppt werden kann, dehydratisiert. Als Katalysatoren eignen sich organische Säuren, wie Sulfonsäuren, beispielsweise Methansulfonsäure und Toluolsulfonsäure oder Ionenaustauscher mit Sulfonsäure- oder Phosphonsäuregruppen. Gemäß einer alternativen Ausführungsform werden als saure Katalysatoren anorganische Feststoffe verwendet, wie sauer wirkende Oxide und Silikate, insbesondere Zeolithe, beispielsweise Zeolith ZSM5, sowie mit einer anorganischen Säure, wie Phosphorsäure oder Anhydriden derselben belegte Oxide, beispielsweise Aluminiumoxid. Geeignet ist zum Beispiel mit Phosphorsäure behandeltes Al₂O₃ das danach kalziniert wurde.

Die Dehydratisierung der Cyanhydrine lässt sich bei deutlich niedrigerer Temperatur durchführen als diese bei der Pyrolyse acylierter Cyanhydrine gemäß US-Patent 2,473,486 erforderlich war. Zweckmäßigerweise erfolgt die Dehydratisierung bei einer Temperatur von unter 150 °C an einem festen Dehydratisierungskatalysator. Bevorzugt wird ein Festbettdehydratisierungskatalysator in Rieselbettfahrweise betrieben, wobei zweckmäßigerweise die Cyanhydrine in einem organischen Lösungsmittel gelöst über den Katalysator geleitet werden.

Gemäß einer bevorzugten Ausführungsform enthält die Cyanhydrine enthaltende Lösung zusätzlich einen Polymerisationsinhibitor, wie Hydrochinon, HydrochinonMonomethylether oder ein tertiär-butyliertes Phenol, in wirksamer Menge.

An das aus der Dehydratisierungsstufe gewonnene Pentadiennitril, das vorzugsweise aus dem Reaktionsgemisch der Dehydratisierung abgetrennt wurde, wird in Gegenwart eines basischen Katalysators Cyanwasserstoff angelagert (Stufe (iv)). Geeignet sind Katalysatoren aus der Reihe tertiärer Amine, Alkalihydroxide, Alkalicyanide, sekundärer und tertiärer Alkaliphosphate und Alkalipyrophosphate. Vorzugsweise erfolgt die HCN-Anlagerung an Pentadiennitril in Gegenwart eines schwach basischen Katalysators, weil in Gegenwart stark basischer Katalysatoren in größerem Umfang außer dem gewünschten cis/trans-1,4-Dicyanobuten-2 und seinem Isomeren cis/trans-1,4-Dicyanobuten-1 durch eine weitere HCN-Anlagerung 1,2,4-Tricyanobutan und weitere Isomere hiervon sowie durch eine Dimerisierung Isomere von Dekatriendinitril und weiteren HCN-Adukten desselben gebildet werden können.

Gemäß einer besonders bevorzugten Ausführungsform der HCN-Addition an cis/trans-Pentadiennitril wird als Katalysator Alkalipyrophosphat, insbesondere Kaliumpyrophosphat oder ein sekundäres Phosphat, insbesondere Dikaliumhydrogenphosphat verwendet. Es wurde festgestellt, dass mit einem derartigen Katalysator, insbesondere Kaliumpyrophosphat, die isomeren cis/trans-1,4-Dicyanobutene in an 100 % heranreichender Selektivität gebildet werden.

Unter der Bezeichnung cis/trans-Pentadiennitril sowie cis/trans-1,4-Dicyanobuten-1 und -2 wird verstanden, dass es sich jeweils um ein Isomerengemisch oder um das jeweilige cis- oder trans-Isomere hiervon handeln kann.

Die in der Stufe (iv) gebildeten cis/trans-1,4-Dicyanobutene-1 und -2, lassen sich in Gegenwart eines an sich bekannten Hydrierkatalysators für Nitrilgruppen und olefinische Doppelbindungen zu Hexamethylendiamin hydrieren (Stufe (v)). Vorzugsweise erfolgt diese Hydrierstufe in Gegenwart eines heterogenen Hydrierkatalysators, mit einem oder mehreren hydrieraktiven Metallen aus der Reihe der 8. Nebengruppe des Periodensystems. Besonders geeignete Katalysatoren enthalten als hydrieraktive Komponente ein oder mehrere Metalle aus der Reihe Kobalt, Nickel, Ruthenium, Palladium und Platin. Der Katalysator kann in Form eines Suspensionskatalysators oder als Festbettkatalysator eingesetzt werden. Der Festbettreaktor wird in Blasenfahrweise oder in Rieselbettfahrweise betrieben. Die Hydrierung erfolgt bei einem Wasserstoffpartialdruck im Bereich von 2 bis 20 MPa, insbesondere 3 bis 10 MPa. Die Hydrierung kann bei einer Temperatur im Bereich von 30 bis etwa 150 °C, insbesondere 70 bis 120 °C durchgeführt werden. Vorzugsweise wird die Hydrierung in Gegenwart eines organischen Lösungsmittels durchgeführt, wobei das Lösungsmittel wiederum einen Polymerisationsinhibitor enthalten kann.

Die erfindungsgemäße Reaktionssequenz zur Herstellung von Hexamethylendiamin aus Butadien umfasst wie das in der Technik genutzte Verfahren auf der Basis von Butadien und Cyanwasserstoff 5 Stufen. Zur Anlagerung von Cyanwasserstoff an das jeweilige olefinische Substrat werden entgegen dem Stand der Technik keine homogenen Nickelkatalysatoren sondern einfache anorganische oder organische Basen verwendet.

Die erfindungsgemäße Reaktionssequenz stellt der Fachwelt eine weitere Möglichkeit zur Herstellung von Hexamethylendiamin aus Butadien zur Verfügung.

Die nachfolgenden Beispiele verdeutlichen das erfindungsgemäße Verfahren.

### Beispiele:

### Vergleichsbeispiel 1 (VB1):

### Herstellung von 1,4-Dicyanobuten gemäß US 2,488,913

Das Beispiel wurde nachgearbeitet. Butadienmonoxid und Cyanwasserstoff wurden im Mol-Verhältnis 1 zu 2 eingesetzt. Das Katalysatorsystem bestand aus CuCl, Cu, HCl (36 %) NH₄Cl und Wasser in den patentgemäßen Mengenverhältnissen. Nach 2h bei 75 - 80 °C wurde das Reaktionsgemisch mit Toluol extrahiert. Gemäß gaschromatographischer Analyse enthielt das von Lösungsmittel befreite Reaktionsgemisch nur 8 Flächen-% 1,4-Dicyanobutene, insbesondere 1,4-Dicyanobuten-2.

### Vergleichsbeispiel 2 (VB2):

### Herstellung gemäß US 4,488,913 (Beispiel 3)

Unter den im US-Patent angegebenen Bedingungen wurde 1,2-Epoxybuten-3 (im Handel erhältlich), in Gegenwart von Calciumhydroxid in wässriger Phase mit Cyanwasserstoff umgesetzt. Nach 2 h wurde kein Umsatz an HCN beobachtet.

### Beispiele 1 bis 11:

### Herstellung von 3-HPN und 2HMBN aus Butadienmonoxid und HCN

1,2-Epoxybuten-3 wurde in Acetonitril (Beispiel 1 bis 3) bzw. Dimehtylformamid (Beispiele 4 bis 11) mit Cyanwasserstoff umgesetzt und zwar 3 h bei 70 °C (Beispiele 1 bis 7) bzw. 3 h bei 120 °C (Beispiele 8 bis 11).

Das Molverhältnis Butadienmonoxid (Bumo) zu Cyanwasserstoff betrug stets 1 zu 1,1. Der Katalysator folgt aus den nachfolgenden Tabellen, die Katalysatoreinsatzmenge betrug 0,5 bis 0,9 Mol% in den Beispielen 1 bis 3 und 0,8 bis 1,2 Mol% in den Beispielen 4 bis 11.

**Tabelle 1:**

| Lösungsmittel Acetonitril, T = 70°C | | | | |
|---|---|---|---|---|
| Bsp. Nr. | Katalysator | Umsatz (%) | Selektivität 3HPN (%) | Regioselektivität (%) 3HPN:2HMBN |
| 1 | LiOH | 7,7 | 35,5 | 91,8 |
| 2 | KOH | 11,3 | 37,8 | 94,0 |
| 3 | JRA400[OH] | 6,3 | 16,4 | 95,7 |
| T = 70 °C; t = 3h; 5 ml Acetonitril; 7,5 mMol Bumo | | | | |

**Tabelle 2:**

| Lösungsmittel DMF, T = 70 °C (B4 - B7), bzw. T = 120 °C (B8 - B11) | | | | |
|---|---|---|---|---|
| Bsp. Nr. | Katalysator | Umsatz (%) | Selektivität 3HPN (%) | Regioselektivität (%) 3HPN:2HMBN |
| 4 | LiOH | 45,7 | 34,7 | 99,6 |
| 5 | LiOH 12,7 % in Wasser | 90,1 | 74,9 | 99,5 |
| 6 | KOH | 43,1 | 28,7 | 99,5 |
| 7 | N-Methylpyrrolindin | 54,8 | 42,7 | 99,9 |
| 8 | LiOH | 93,9 | 78,5 | 99,4 |
| 9 | KOH | 88,9 | 74,1 | 99,1 |
| 10 | JRA400[OH] | 87,3 | 71,7 | 99,2 |
| 11 | Ca(OH)₂ | 81,6 | 69,9 | 98,7 |

### Beispiel 12: Dehydratisierung von 3HPN zu Pentadiennitril

3HPN wurde in toluolischer Lösung in Gegenwart von p-Toluolsulfonsäure unter Rückfluss gekocht und gebildetes Wasser azeotrop abgetrennt. Es bildet sich Pentadiennitril (Gemisch aus dem cis und trans Isomeren).

### Beispiel 13:

### Herstellung von 1,4-Dicyanobutenen (DCB) durch HCN-Addition an Pentadiennitril (PDN)

Pentadiennitril und HCN wurden in Acetonitril bei einem Molverhältnis HCN:PDN: Katalysator von 1:1,015:(0,01 - 0,1) umgesetzt.

**Tabelle 3:**

| Bsp. Nr. | Katalysator | Umsatz PDN(%) | Selektivität DCB (%) | T (°C) | t (h) |
|---|---|---|---|---|---|
| 13^{*)} | LiOH | 58,7 | 39,9 | 40 | 64 |
| 14 | Triethylamin | 16,6 | 65,6 | 40 | 64 |
| 15 | N-Methylpyrrolidin | 20 | 59,6 | 40 | 64 |
| 16 | Kaliumpyrophosphat | 24,5 | 98,8^{**)} | 80 | 2, |
| | | | | 25 | dann 22, |
| | | | | 80 | dann 2 |
| 17 | N-Methylpyrrolidin | 10,0 | 53,2 | 80 | 2, |
| | | | | 25 | dann 22, |
| | | | | 80 | dann 2 |

| | | | | | |
|---|---|---|---|---|---|
| ^{*)} Ausbeute an DCB 23,4 %, zusätzlich entstanden 22,5 % Nebenprodukte, insbesondere Tricyanobutan + Tricyanooctadien-Isomere | | | | | |
| ^{**)} Gebildet wurden ca. 80 % cis/trans-DCB-2 und 20 % cis/trans-DCB-1 | | | | | |

## Patentansprüche

1. Verfahren zur Herstellung von Hexamethylendiamin aus Butadien,
**gekennzeichnet durch** die hintereinander durchgeführten Stufen (i) einer katalytischen Epoxidation von Butadien zu 1,2-Epoxy-3-buten (Butadienmonoxid), (ii) einer basisch katalysierten Addition von Cyanwasserstoff an Butadienmonoxid unter Bildung eines 3-Hydroxy-4-pentennitril (3HPN) und 2-Hydroxymethyl-3-butennitril (2HMBN)enthaltenden Reaktionsgemischs , (iii) einer sauer katalysierten Dehydratisierung der Cyanhydrine 3HPN und 2HMBN der Stufe (ii) zu cis/trans-Pentadiennitril (PDN), (iv) einer basisch katalysierten Addition von Cyanwasserstoff an cis/trans-Pentadiennitril der Stufe (iii) unter Bildung von cis/trans-1,4-Dicyanobuten-1 und -2 (DCB) und (v) einer katalytischen Hydrierung der isomeren cis/trans-1,4-Dicyanobutene der Stufe (iv) zu Hexamethylendiamin.

2. Verfahren nach Anspruch 1,
**dadurch gekennzeichnet,**
**dass** man die Cyanwasserstoffaddition in der Stufe (ii) in Gegenwart eines Katalysators durchführt, ausgewählt aus der Reihe der Alkalihydroxide, Alkalicyanide, Erdalkalihydroxide, ausgenommen Calciumhydroxid in wässriger Phase, Erdalkalicyanide, basischen Ionenaustauscher in der OH-Form und aliphatischen und carbocyclischen sekundären oder tertiären Amine.

3. Verfahren nach Anspruch 2,
**dadurch gekennzeichnet,**
**dass** man als Katalysator Lithiumhydroxid, in-situ gebildetes Lithiumcyanid oder ein N-alkyliertes cyclisches Amin mit 5 oder 6 Ringgliedern, wobei Alkyl für Methyl, Ethyl, n-Propyl oder n-Butyl steht und der Ring zusätzlich ein weiteres Heteroatom enthält, verwendet.

4. Verfahren nach einem oder mehreren der Ansprüche 1 bis 3,
**dadurch gekennzeichnet,**
**dass** man die Cyanwasserstoffaddition in der Stufe (ii) in Gegenwart eines aprotischen dipolaren Lösungsmittels, insbesondere Acetonitril, N-Methylpyrrolidon oder Dimethylformamid, durchführt.

5. Verfahren nach einem oder mehreren der Ansprüche 1 bis 4,
**dadurch gekennzeichnet,**
**dass** man in der Stufe (ii) Butadienmonoepoxid gegenüber Cyanwasserstoff im Überschuss einsetzt.

6. Verfahren nach einem oder mehreren der Ansprüche 1 bis 5,
**dadurch gekennzeichnet,**
**dass** man die Dehydratisierung der Cyanhydrine der Stufe (iii) in Gegenwart eines sauren Festbettkatalysators durchführt.

7. Verfahren nach einem oder mehreren der Ansprüche 1 bis 6,
**dadurch gekennzeichnet,**
**dass** man die Dehydratisierung in Gegenwart eines Polymerisationsinhibitors durchführt.

8. Verfahren nach einem oder mehreren der Ansprüche 1 bis 7,
**dadurch gekennzeichnet,**
**dass** man die Cyanwasserstoffaddition an cis/trans-Pentadiennitril der Stufe (iv) in Gegenwart eines Katalysators aus der Reihe der tertiären Amine, Alkalihydroxide, Alkalicyanide, sekundären und tertiären Alkaliphosphate und Alkalipyrophosphate durchführt.

9. Verfahren nach Anspruch 8,
**dadurch gekennzeichnet,**
**dass** man als Katalysator ein Alkalipyrophosphat, insbesondere Kaliumpyrophosphat, oder ein sekundäres Phosphat, insbesondere Dikaliumhydrogenphosphat, verwendet.

10. Verfahren nach einem oder mehreren der Ansprüche 1 bis 9,
**dadurch gekennzeichnet,**
**dass** man die Hydrierung des cis/trans-Dicyanobutene zu Hexamethylendiamin (Stufe (v)) in Gegenwart eines heterogenen Hydrierungskatalysators mit einem oder mehreren hydrieraktiven Metallen aus der Reihe der 8. Nebengruppe des Periodensystems, insbesondere aus der Reihe Co, Ni, Ru, Pd und Pt, durchführt.

11. Verfahren zur Herstellung von cis/trans-1,4-Dicyanobutenen, insbesondere 1,4-Dicyanobuten-2, durch Anlagerung von Cyanwasserstoff an cis/trans-Pentadiennitril,
**dadurch gekennzeichnet,**
**dass** man die Anlagerung in Gegenwart eines Katalysators aus der Reihe der Alkalipyrophosphate und Dialkalihydrogenphosphate durchführt.

12. Verfahren zur Herstellung von Hexamethylendiamin aus 1,2-Epoxy-3-buten, umfassend die Addition von Cyanwasserstoff an 1,2-Epoxy-3-buten in Gegenwart eines basischen Katalysators und katalytische Hydrierung der als Zwischenprodukt gebildeten 1,4-Dicyanobutene,
**dadurch gekennzeichnet,**
**dass** man die Cyanwasserstoffaddition an 1,2-Epoxy-3-buten in Gegenwart eines Katalysators, ausgewählt aus der Reihe der Alkalihydroxide, Alkalicyanide, Erdalkalihydroxide, ausgenommen Calciumhydroxid in wässriger Phase, Erdalkalicyanide, basischen Ionenaustauscher in der OH-Form und aliphatischen und carbocyclischen sekundären oder tertiären Amine durchführt, wobei ein 3-Hydroxy-4-pentennitril (3-HPN) und 2-Hydroxymethyl-3-butennitril (2HMBN) enthaltendes Reaktionsgemisch gebildet wird, sauer katalysierte Dehydratisierung der Cyanhydrine 3HPN und 2HMBN unter Bildung von cis/trans-Pentadiennitril (PDN), basisch katalysierte Addition von Cyanwasserstoff an cis/trans-Pentadiennitril unter Bildung von cis/trans-1,4-Dicyanobutenen, insbesondere cis/trans-1,4-Dicyanobuten-2.

13. Verfahren nach Anspruch 12,
**dadurch gekennzeichnet,**
**dass** man als Katalysator für die HCN-Addition an 1,2-Epoxybuten-3 Lithiumhydroxid, in-situ gebildetes Lithiumcyanid oder ein N-alkyliertes cyclisches Amin mit 5 oder 6 Ringgliedern, wobei Alkyl für Methyl, Ethyl, n-Propyl oder n-Butyl steht und der Ring zusätzlich ein weiteres Heteroatom enthalten kann, verwendet.

14. Verfahren nach Anspruch 12 oder 13,
**dadurch gekennzeichnet,**
**dass** man als Katalysator für die HCN-Addition an cis/trans-Pentadiennitril ein Alkalipyrophosphat, insbesondere Kaliumpyrophosphat, oder ein sekundäres Phosphat, insbesondere Dikaliumhydrogenphosphat, verwendet.
